(19) Europäisches Patentamt
European Patent Office
Office européen des brevets

(11) Veröffentlichungsnummer : **0 388 870 B1**

(12) **EUROPÄISCHE PATENTSCHRIFT**

(45) Veröffentlichungstag der Patentschrift : **09.06.93 Patentblatt 93/23**

(51) Int. Cl.⁵ : **C07C 25/13,** C07C 17/22

(21) Anmeldenummer : **90105195.3**

(22) Anmeldetag : **20.03.90**

# (54) Verfahren zur Herstellung von aliphatischen substituierten Fluorbenzolen.

(30) Priorität : **22.03.89 DE 3909486**

(43) Veröffentlichungstag der Anmeldung : **26.09.90 Patentblatt 90/39**

(45) Bekanntmachung des Hinweises auf die Patenterteilung : **09.06.93 Patentblatt 93/23**

(84) Benannte Vertragsstaaten : **AT BE CH DE FR GB IT LI NL**

(56) Entgegenhaltungen :
**EP-A- 0 330 420**
**DD-A- 2 365 18**
**DE-B- 2 732 604**

(56) Entgegenhaltungen :
**THE JOURNAL OF ORGANIC CHEMISTRY; Band 26. 1961, Easton, USA; E. D. BERGMANN et al: "2,3-Difluorostyrene and 2-chloro-5-fluorostyrene. The Preparation of aromatic fluorine compounds", Seiten 919-923**

(73) Patentinhaber : **BASF Aktiengesellschaft**
**Carl-Bosch-Strasse 38**
**W-6700 Ludwigshafen (DE)**

(72) Erfinder : **Wild, Jochen, Dr.**
**An der Marlach 7**
**W-6705 Deidesheim (DE)**
Erfinder : **Harreus, Albrecht, Dr.**
**Teichgasse 13**
**W-6700 Ludwigshafen (DE)**
Erfinder : **Goetz, Norbert, Dr.**
**Schoefferstrasse 25**
**W-6520 Worms 1 (DE)**

EP 0 388 870 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von aliphatisch substituierten Fluorbenzolen der allgemeinen Formel I

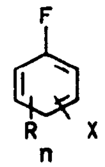

I

in der
R für einen aliphatischen oder cycloaliphatischen Rest steht,
n eine der Zahlen 2, 3 oder 4 bedeutet,
und in der
X ein Wasserstoff-, Fluor-, Chlor- oder Bromatom ist,
durch die Diazotierung der entsprechenden Anilinderivate der allgemeinen Formel II

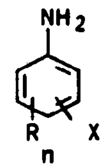

II

in Gegenwart von Tetrafluoroborsäure und Zersetzung der erhaltenen Diazoniumtetrafluoroborate der allgemeinen Formel III

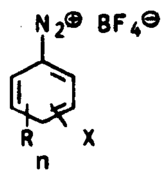

III

Zur Synthese der Alkylfluorbenzole der allgemeinen Formel I wird im allgemeinen so vorgegangen, daß aus den Anilinderivaten der Formel II sukzessive durch Diazotierung die Diazoniumtetrafluoroborate III erzeugt und diese, gegebenenfalls nach Isolierung und Trocknung, thermisch zu den Fluorbenzolen I zersetzt werden. Diese Vorgehensweise samt ihrer Varianten ist als Balz-Schiemann-Reaktion bekannt (Übersichten dazu: Roe, Organic reactions, Vol. V, Chapter 4; Houben-Weyl, Methoden der organischen Chemie, Bd. 5/3, S. 213-245, Thieme, Stuttgart 1962; Schiemann/Cornils: Chemie und Technologie cyclischer Fluorverbindungen, S. 3-25, Enke, Stuttgart 1969; Hudlicky: Chemistry of Organic Fluorine Compounds, S. 160-169, Ellis Horwood, Chichester 1976).

Eine Variante der Balz-Schiemann-Reaktion, bei der die Zersetzung der Diazoniumtetrafluoroborate III durch Kupferpulver oder Kupfersalze katalysiert wird, wurde von Bergmann et al. (J. Am. Chem. Soc. 78, 6037 (1956); J. Org. Chem. 19, 1594 (1954); J. Org. Chem. 26, 919 (1961)) eingeführt. Dabei werden zunächst durch Diazotierung die Diazoniumtetrafluoroborate III hergestellt, isoliert und in wäßrigem Aceton oder, falls die Diazoniumtetrafluoroborate im Medium der Diazotierungsreaktion löslich sind, in dieser Lösung durch Zugabe der Kupferkatalysatoren zersetzt.

Obwohl die Bergmann-Variante gegenüber der herkömmlichen Balz-Schiemann-Reaktion eine Reihe von Vorteilen hat, beispielsweise wird durch den Kupferzusatz die Zersetzungstemperatur der Diazoniumsalze deutlich erniedrigt, läßt sie sich nicht wirtschaftlich in den technischen Maßstab übertragen, und zwar aus mehreren Gründen:

1. Die Isolierung der Diazoniumtetrafluoroborate III aus dem Medium der Diazotierungsreaktion und die gesonderte Zersetzung dieser Salze ist unwirtschaftlich.

2. Falls die Diazoniumtetrafluoroborate III schwer lösliche Verbindungen sind, und dies ist meistens der Fall, erhält man schwer rührbare, hochviskose Suspensionen dieser Salze. Zur Aufrechterhaltung oder Wiederherstellung der Rührbarkeit dieser Suspensionen muß mit großen Lösungsmittelmengen gearbeitet werden, wodurch das Verfahren stark verteuert wird: Zum einen wird, bedingt durch den Einsatz großer Lösungsmittelmengen, die Raum-Ausbeute erniedrigt und der Einsatz großer, kostenintensiver Reaktoren erforderlich, zum anderen müssen große Lösungsmittelmengen aufgearbeitet oder entsorgt werden.

3. Bei der Zersetzung der Diazoniumtetrafluoroborate entstehen relativ große Mengen an Stickstoff und gasförmigem Bortrifluorid. Wird die Zersetzung der Diazoniumsalze in Suspension oder in Lösung durchgeführt, bewirkt diese Gasentwicklung ein Aufschäumen des Reaktionsmediums und dadurch dessen starke Volumenvergrößerung, wodurch ebenfalls die Verwendung unwirtschaftlich großer Reaktoren erforderlich wird. Da der Zusatz der Kupferkatalysatoren eine schlagartige Zersetzung der gesamten Diazoniumtetrafluoroborate zur Folge haben kann, besteht bei dieser Verfahrensweise weiterhin die latente Gefahr des unkontrollierbaren Aufschäumens. Zur Minderung dieser Gefahr kann das Verfahren nur mit geringen Raum-Zeit-Durchsätzen betrieben und daher nur mit hohen Kosten ausgeführt werden.

Da Alkylfluorbenzole I wichtige Zwischenprodukte zur Herstellung von Pflanzenschutzmitteln sind, bestand die Aufgabe, ein Verfahren zur wirtschaftlichen und sicherheitstechnisch unbedenklichen Herstellung von Alkylfluorbenzolen zu finden.

Dementsprechend wurde ein Verfahren zur Herstellung von aliphatisch substituierten Fluorbenzolen der allgemeinen Formel I

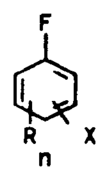

I

in der

R für einen aliphatischen oder cycloaliphatischen Rest steht,
n eine der Zahlen 2, 3 oder 4 bedeutet,
und in der
X ein Wasserstoff-, Fluor-, Chlor- oder Bromatom ist,
durch die Diazotierung der entsprechenden Anilinderivate der allgemeinen Formel II

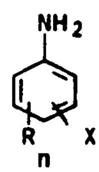

II

in Gegenwart von Tetrafluoroborsäure und Zersetzung der erhaltenen Diazoniumtetrafluoroborate der allgemeinen Formel III

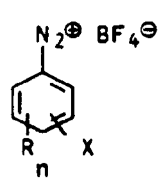

III

gefunden, das dadurch gekennzeichnet ist, daß die Zersetzungsreaktion einhergehend mit der Diazotierungsreaktion, in Gegenwart von elementarem Kupfer und/oder Kupfer(I)- und/oder Kupfer(II)-Salzen und bei Temperaturen von -15 bis 80°C durchgeführt wird.

Die nach dem erfindungsgemäßen Verfahren vorteilhaft herstellbaren Alkylfluorobenzole I können mit 2, 3 oder 4 $C_1$- bis $C_8$-Alkyl, $C_2$- bis $C_8$-Alkenyl, $C_3$- bis $C_8$-Cycloalkyl und/oder $C_5$- bis $C_8$-Cycloalkenylgruppen

R und gegebenenfalls mit einem Fluor-, Chlor- oder Bromatom X substituiert sein.

Im erfindungsgemäßen Verfahren zur Herstellung der Alkylfluorbenzole I werden die Diazotierungs- und die Zersetzungsreaktion nebeneinander in Gegenwart von elementarem Kupfer und/oder Kupfer(I)- und/oder Kupfer(II)-Salzen durchgeführt. Überraschenderweise hat diese Maßnahme zur Folge, daß beim erfindungsgemäßen Verfahren die oben geschilderten Nachteile der bislang bekannten Verfahren vermieden werden.

Dabei kann die Diazotierung der Anilinderivate II zu den Diazoniumtetrafluoroboraten III auf an sich bekannte Weise erfolgen, indem das Anilinderivat im sauren Milieu mit zweckmäßigerweise stöchiometrischen Mengen eines organischen, vorzugsweise eines anorganischen, insbesondere eines Alkalimetallnitrits, wie Natriumnitrit, umgesetzt wird. Da zur Herstellung der Alkylfluorbenzole I die Tetrafluoroborsäuresalze der entsprechenden Diazoniumverbindungen benötigt werden, wird das zur Diazotierung erforderliche, saure Milieu vorteilhaft mit wäßriger Tetrafluoroborsäure erzeugt. Die Konzentration der wäßrigen Tetrafluoroborsäurelösung ist im allgemeinen nicht kritisch, zweckmäßigerweise verwendet man jedoch 1 bis 60, vorzugsweise 30 bis 60 gew.-%ige wäßrige Tetrafluoroborsäurelösungen.

Erfindungsgemäß werden im vorliegenden Verfahren die Diazotierungs- und die Zersetzungsreaktion nebeneinander, in einer Eintopfreaktion vorgenommen, d.h. die Diazoniumtetrafluoroborate werden in dem Maße, wie sie gebildet werden, zu den Alkylfluorbenzolen zersetzt. Dadurch wird die Anreicherung der Diazoniumtetrafluoroborate im Reaktionsmedium mit den beschriebenen, negativen Auswirkungen vermieden. Um eine glatte Zersetzung der Diazoniumtetrafluoroborate zu gewährleisten, wird die Diazotierungsreaktion deshalb in Gegenwart kupferhaltiger Zersetzungskatalysatoren durchgeführt.

Als Zersetzungskatalysatoren können elementares Kupfer, vorzugsweise elementares Kupfer mit großer Oberfläche, z.B. Kupferpulver, als auch Kupfer(I)- und/oder Kupfer(II)-Salze verwendet werden. Die Art der Kupfersalze ist im allgemeinen nicht kritisch, beispielsweise können Kupfer(I)- und/oder Kupfer(II)-Nitrate, Acetate, Halogenide usw. verwendet werden, bevorzugt finden Kupfer(II)-Sulfat, Kupfer(II)-Tetrafluoroborat sowie Kupfer(I)- und/oder Kupfer(II)-Fluorid Anwendung. Die Anwendung anderer Kupferhalogenide kann in geringem Maße zur Bildung von am Benzolkern halogenierten Nebenprodukten führen. Die Verwendung von Katalysatormischungen aus verschiedenerlei Kupfersalzen und gegebenenfalls elementarem Kupfer ist selbstverständlich ebenfalls möglich.

Die kupferhaltigen Zersetzungskatalysatoren werden im allgemeinen in Mengen von 0,1 bis 20 mol-%, bezogen auf das Anilinderivat II, der Reaktionsmischung zugesetzt. Bei geringeren Katalysatormengen erhöht sich der Zeitbedarf für die Zersetzungsreaktlon und bei größeren Mengen werden im allgemeinen keine nennenswerten Vorteile mehr erzielt.

Die Umsetzung der Anilinderivate II zu den Fluorbenzolen I wird im allgemeinen bei Temperaturen von -15 bis 80°C, vorteilhaft bei 0 bis 60°C, durchgeführt. Dabei wird die Temperatur der gesamten Umsetzung, also der Diazotierungs- und der Zersetzungsreaktion, vorzugsweise so gewählt, daß sie 5 bis 15°C oberhalb der Zersetzungstemperatur der jeweiligen Diazoniumtetrafluoroborate, bei Anwesenheit der jeweiligen Kupferkatalysatoren, liegt. Es versteht sich von selbst, daß die Zersetzungstemperatur der Diazoniumtetrafluoroborate III von Verbindung zu Verbindung variiert und für die Einzelverbindungen erforderlichenfalls ermittelt werden muß, im allgemeinen zersetzen sich die von den Anilinderivaten II abgeleiteten Diazoniumsalze III jedoch im Bereich von 10 bis 50°C.

Sowohl die Diazotierung der Anilinderivate II als auch die Zersetzung der Diazoniumtetrafluoroborate III werden zweckmäßigerweise in wäßriger Tetrafluoroborsäure als Lösungsmittel vorgenommen. In Folge der erfindungsgemäßen Reaktionsführung kann die Menge der sowohl als Reagenz als auch als Lösungsmittel dienenden Tetrafluoroborsäure gering gehalten werden - in der Regel sind nicht mehr als 300 ml wäßrige Tetrafluoroborsäurelösung pro Mol Anilinderivat II erforderlich. Die Anwendung größerer Mengen an Tetrafluoroborsäure ist möglich, führt aber zu keinen weiteren Vorteilen. Der Zusatz von unter den Reaktionsbedingungen inerten, wasserlöslichen, organischen Lösungsmitteln wie Dioxan oder Tetrahydrofuran zum wäßrigen Reaktionsmedium ist ebenfalls möglich und kann sich im Einzelfall vorteilhaft auswirken.

Bei der Durchführung der Umsetzung wird üblicherweise so vorgegangen, daß das Anilinderivat II zusammen mit der Tetrafluoroborsäure und dem Kupferkatalysator im Reaktionsgefäß im allgemeinen bei der Reaktionstemperatur vorgelegt und dazu das Nitrit, zweckmäßigerweise in gelöster Form, dosiert wird. Das Verfahren kann in herkömmlichen Reaktoren sowohl in der diskontinuierlichen als auch in der kontinuierlichen Betriebsweise ausgeübt werden. Die Isolierung der Alkylfluorbenzole aus dem Reaktionsgemisch kann nach herkömmlichen Methoden wie Extraktion, Destillation oder Kristallisation erfolgen.

Überraschenderweise lassen sich nach dem erfindungsgemäßen Verfahren die Alkylfluorbenzole I im industriellen Maßstab auf kostengünstige, einfache und bequeme Weise und im allgemeinen ohne nennenswerte Verunreinigungen durch phenolische Nebenprodukte erhalten.

Die als Ausgangsmaterial dienenden Anilinderivate II sind bekannt oder nach bekannten Verfahren erhältlich (s. z.B. EP-A-53 696; US-A-3 678 113; DE-A-33 09 354; bzw. Izv. Akad. Nauk. SSR, Ser. Khim. 31, 2160

(1982)).

Die Alkylfluorbenzole I finden als Zwischenprodukte zur Herstellung von Pflanzenschutzmitteln, beispielsweise Pyrethroiden, wie sie in O.Z. EP-A 347 694 beschrieben sind, Verwendung. Dazu werden sie, beispielsweise durch elektrochemische Oxidation (s. O.Z. EP-A 347 690) in die entsprechenden kernfluorierten Benzylalkohole überführt, welche dann, beispielsweise mit Chrysanthemumsäurederivaten, verestert werden.

## Beispiele

### Beispiel 1

Herstellung von 2-Isopropyl-6-methylfluorbenzol

1,4 l 40 %ige wäßrige Tetrafluoroborsäure wurden bei 0°C mit 298 g (2 mol) 2-Isopropyl-6-methylanilin und 26 g Kupferpulver (0,4 mol) versetzt. Anschließend wurde bei 25°C unter Kühlung eine Lösung aus 138 g (2 mol) Natriumnitrit in 550 ml Wasser zugetropft und die Reaktionsmischung 12 Stunden lang bei Raumtemperatur gerührt. Zur Aufarbeitung wurde die Reaktionsmischung in 3 l Eiswasser gegossen und das Produkt aus dieser Mischung mit Methylenchlorid extrahiert. Die organische Phase wurde über Natriumsulfat getrocknet, eingeengt und das so erhaltene Rohprodukt über eine 15 cm Füllkörperkolonne bei 146 mbar fraktionierend destilliert.
Ausbeute: 73 %.
Nebenprodukt (2-Isopropyl-6-methylphenol): 3,3 %

### Beispiel 2

Die Umsetzung wurde wie in Beispiel 1 beschrieben durchgeführt. Anstelle von Kupferpulver wurden 25 g Kupfer(II)sulfat (0,1 mol) verwendet.
Ausbeute 72 % d. Th.
Nebenprodukt (2-Isopropyl-6-methylphenol): 2,7 %.

### Beispiel 3

Die Umsetzung wurde wie in Beispiel 1 beschrieben durchgeführt. Anstelle von Kupferpulver wurden 50 g Kupfer(II)bis-tetrafluoroborat (0,2 mol) eingesetzt.
Ausbeute: 66 % d. Th.
Nebenprodukt (2-Isopropyl-6-methylphenol): 4 %.

Nach dem Verfahren von Beispiel 1 wurden die Fluorbenzole der Beispiele 4 bis 7 hergestellt. Die Ergebnisse dieser Experimente sind in Tabelle 1 aufgelistet.

Tabelle:

| Beispiel Nr. | Verbindung | Ausbeute | Phenolbildung |
|---|---|---|---|
| 4 | 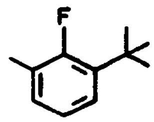 | 49 % | 6,7 % |
| 5 | 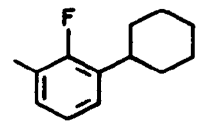 | 75 % | 2,5 % |
| 6 | 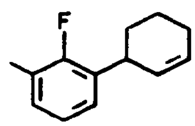 | 77 % | 2,9 % |
| 7 | 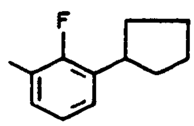 | 74 % | 3,5 % |

## Patentansprüche

1. Verfahren zur Herstellung von aliphatisch substituierten Fluorbenzolen der allgemeinen Formel I

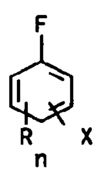

I

in der
R für einen aliphatischen oder cycloaliphatischen Rest steht,
n eine der Zahlen 2, 3 oder 4 bedeutet,
und in der
X ein Wasserstoff-, Fluor-, Chlor- oder Bromatom ist,
durch die Diazotierung der entsprechenden Anilinderivate der allgemeinen Formel II

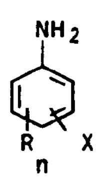

II

in Gegenwart von Tetrafluoroborsäure und Zersetzung der erhaltenen Diazoniumtetrafluoroborate der allgemeinen Formel III

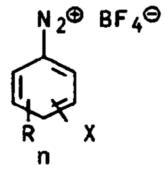

III

dadurch gekennzeichnet, daß die Zersetzungsreaktion einhergehend mit der Diazotierungsreaktion, in Gegenwart von elementarem Kupfer und/oder Kupfer(I)- und/oder Kupfer(II)-Salzen und bei Temperaturen von -15 bis 80°C durchgeführt wird.

**2.** Verfahren nach Anspruch 1, dadurch gekennzeichnet, daß die Temperatur der gesamten Umsetzung so gewählt wird, daß sie 5 bis 15°C oberhalb der Zersetzungstemperatur des Diazoniumtetrafluoroborates III bei Anwesenheit von Kupferkatalysatoren liegt.

**3.** Verfahren nach den Ansprüchen 1 oder 2, dadurch gekennzeichnet, daß die Umsetzung in wäßrigem Medium durchgeführt wird.

**4.** Verfahren nach den Ansprüchen 1 bis 3, dadurch gekennzeichnet, daß der Rest R für eine $C_1$- bis $C_8$-Alkyl-, $C_2$- bis $C_8$-Alkenyl-, $C_3$- bis $C_8$-Cycloalkyl- und/oder für eine $C_5$- bis $C_8$-Cycloalkenylgruppe steht.

**Revendications**

**1.** Procédé de fabrication de fluorobenzènes à substitutions aliphatiques de formule générale I

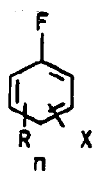

I

dans laquelle
R est mis pour un reste aliphatique ou cycloaliphatique,
n est l'un des nombres 2, 3 ou 4,
et dans laquelle
X est un atome d'hydrogène, de fluor, de chlore ou de brome,
par diazotation du dérivé d'aniline correspondant de formule générale II

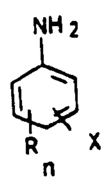

II

en présence d'acide tétrafluoroborique et par décomposition du tétrafluoroborate de diazonium obtenu de formule générale III

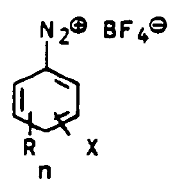

III

caractérisé en ce que la réaction de décomposition accompagnant la réaction de diazotation, est effectuée

à des températures comprises entre -15 et 80°C, en présence de cuivre élémentaire et/ou de sels de cuivre(I) et/ou de cuivre(II).

2. Procédé selon la revendication 1, caractérisé en ce que la température de la conversion totale est choisie de telle manière qu'elle se situe 5 à 15°C en-dessous de la température de décomposition du tétrafluoroborate de diazonium III en présence de catalyseurs de cuivre.

3. Procédé selon la revendication 1 ou 2, caractérisé en ce que la conversion est effectuée en milieu aqueux.

4. Procédé selon l'une des revendications 1 à 3, caractérisé en ce que le reste R est mis pour un groupe alkyle en $C_1$-$C_8$, alcényle en $C_2$-$C_8$, cycloalkyle en $C_3$-$C_8$ et/ou pour un groupe cycloalcényle en $C_5$-$C_8$.

## Claims

1. A process for the preparation of an aliphatically substituted fluorobenzene of the formula I

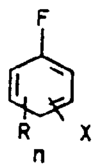

I

where R is an aliphatic or cycloaliphatic radical, n is 2, 3 or 4 and X is hydrogen, fluorine, chlorine or bromine, by diazotization of the corresponding aniline derivative of the formula II

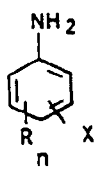

II

in the presence of tetrafluoboric acid and decomposition of the resulting diazonium tetrafluoborate of the formula III

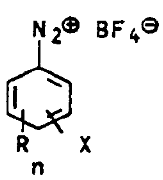

III

wherein the decomposition reaction is carried out simultaneously with the diazotization reaction, in the presence of elemental copper and/or copper(I) and/or copper(II) salts and at from -15 to 80°C.

2. A process as claimed in claim 1, wherein the temperature of the total reaction is chosen so that it is from 5 to 15°C above the decomposition temperature of the diazonium tetrafluoborate III in the presence of copper catalysts.

3. A process as claimed in claim 1 or 2, wherein the reaction is carried out in an aqueous medium.

4. A process as claimed in any of claims 1 to 3, wherein R is $C_1$-$C_8$-alkyl, $C_2$-$C_8$-alkenyl, $C_3$-$C_8$-cycloalkyl and/or $C_5$-$C_8$-cycloalkenyl.